# FASCICULE DE BREVET EUROPEEN

(11) **EP 0 444 989 B1**
(45) Date de publication et mention de la délivrance du brevet: **14.09.1994**
(21) Numéro de dépôt: 91400360.3
(22) Date de dépôt: 13.02.1991
(51) Int. Cl.: C07C 15/16, C07C 2/86, H01B 3/22

(54) **Compositions diélectriques à base de benzyltoluène et de (méthylbenzyl)xylène**
Benzyltoluol und (Methylbenzyl)xylol enthaltende dielektrische Zusammensetzungen
Dielectric compositions comprising benzyltoluene and (methylbenzyl)xylene

(30) Priorité: 27.02.1990 FR 9002420
(43) Date de publication de la demande: 04.09.1991
(73) Titulaire: ELF ATOCHEM S.A., 92800 Puteaux (FR)
(72) Inventeur: Berger, Noelle, F-69130 Ecully (FR); Commandeur, Raymond, F-38220 Vizille (FR); Jay, Pierre, F-69370 Saint-Didier au Mont d'Or (FR)

(56) Documents cités:
- EP-A- 0 136 230
- EP-A- 0 299 867

## Description

La présente invention concerne des compositions diélectriques à base de benzyltoluène et de (méthylbenzyl)xylène.

Le brevet européen EP 136 230 décrit des diélectriques constitués essentiellement d'un mélange d'isomères du benzyltoluène et du dibenzyltoluène et pouvant contenir aussi du ditolylphénylméthane.

La demanderesse a maintenant trouvé qu'en mélangeant ces produits avec du (méthylbenzyl)xylène ou des oligomères du (méthylbenzyl)xylène, on obtient des diélectriques présentant un point de cristallisation particulièrement bas. Cette propriété est particulièrement importante pour les liquides diélectriques utilisés dans les transformateurs de tension ou les condensateurs de puissance installés à l'extérieur.

La présente invention est donc une nouvelle composition caractérisée en ce qu'elle comprend au moins un oligomère du benzyltoluène et au moins un oligomère du (méthylbenzyl)xylène.

On appelle oligomère du benzyltoluène un isomère ou un mélange d'isomères de formule A1 : 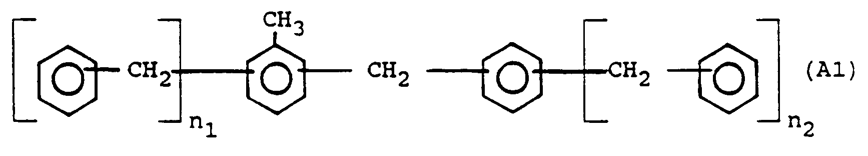
avec n₁ et n₂ = 0, 1 ou 2 sachant que n₁ + n₂ est inférieur ou égal à 3.

On appelle oligomère du (méthylbenzyl)xylène, un isomère ou un mélange d'isomères de formule A2 : 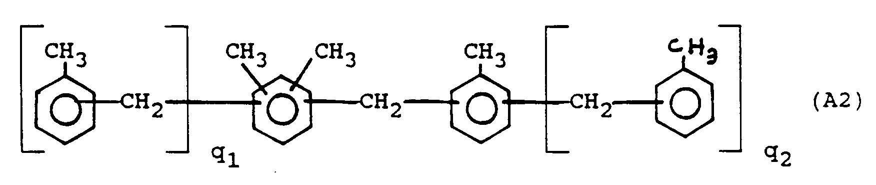
avec q₁ et q₂ = 0, 1 ou 2 sachant que q₁ + q₂ est inférieur ou égal à 3.

On désigne par benzyltoluène l'oligomère A1 dans lequel n₁ + n₂ = 0, et par dibenzyltoluène l'oligomère A1 dans lequel n₁ + n₂ = 1. On désigne par (méthylbenzyl)xylène l'oligomère A2 dans lequel q₁ + q₂ = 0, et par di(méthylbenzyl)xylène l'oligomère A2 dans lequel q₁ + q₂ = 1.

La composition selon l'invention peut donc comprendre du benzyltoluène, du dibenzyltoluène et du di(méthylbenzyl)xylène. Un autre composition selon l'invention peut être du di(méthylbenzyl)xylène, du produit A2 dans lequel n₁ + n₂ = 3 et du benzyltoluène ou toute autre combinaison pourvu qu'il y ait au moins un produit A1 et au moins un produit A2. Selon une autre forme de l'invention, l'oligomère A1 peut être accompagné d'un oligomère B1 qui est un isomère ou un mélange d'isomères de formule : 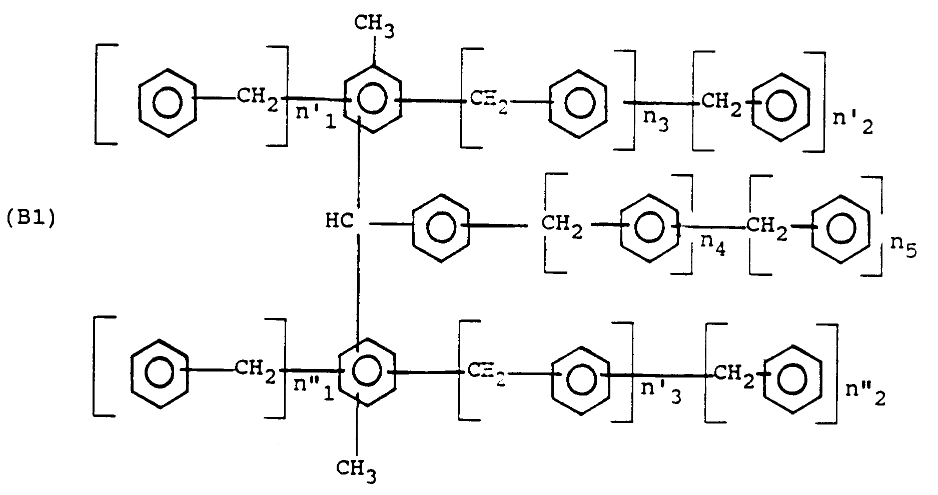
dans laquelle n'₁, n''₁ et n₄ valent 0, 1 ou 2
n'₂, n''₂, n₃, n'₃ et n₅ valent 0 et 1
sachant que n'₁ + n''₁ + n'₂ - n''₂ + n₃ + n'₃ + n₄ + n₅, qu'on désigne par S₁ₙ est inférieur ou égal à 2.

De même l'oligomère A2 peut être accompagné d'un oligomère B2 qui est un isomère ou un mélange d'isomères de formule : 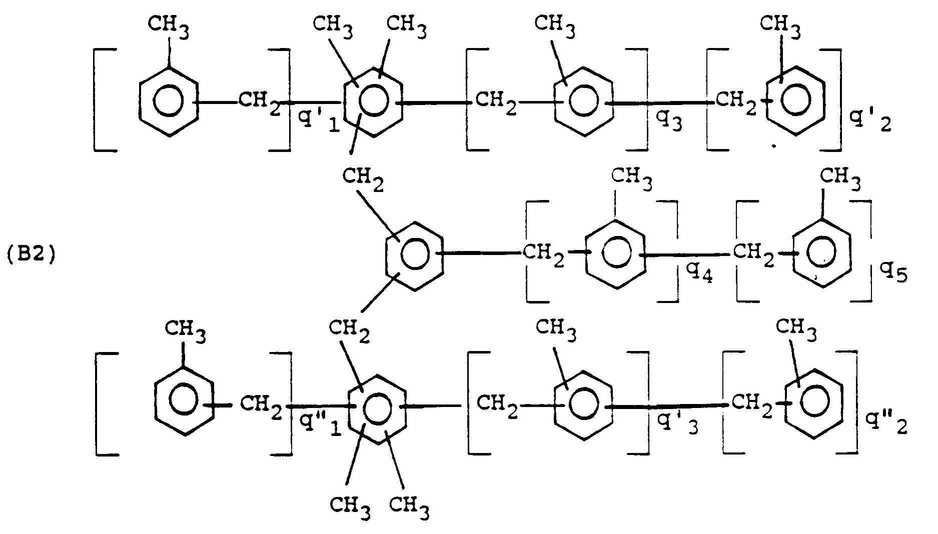
dans laquelle q'₁, q''₁ et q₄ valent 0, 1 ou 2
q'₂, q''₂, q₃, q'₃ et q₅ valent 0 et 1
sachant que q'₁ + q''₁ + q'₂ + q''₂ + q₃ + q'₃ + q₄ + q₅, qu'on désigne par S_{2q} est inférieur ou égal à 2.

On utilise avantageusement des compositions ayant au moins 10 parties de produit A2 pour 90 parties de produits A1.

De préférence, la quantité de A2 est de 15 à 90 parties pour respectivement 85 à 10 parties de produit A1.

Selon une autre forme préférée de l'invention, la répartition des produits de la famille A1 (en % poids) varie de :
60 à 90 % pour les isomères A1 ayant n₁ + n₂ = 0
5 à 40 % pour les isomères A1 ayant n₁ + n₂ = 1
0,5 à 8 % pour les isomères A1 ayant n₁ + n₂ = 2
Le total étant 100 %.

Il en est de même pour les produits de la famille A2 .

La quantité de produits B1 est avantageusement au plus de 15 parties pour 100 parties de A1, et de préférence de 2 à 6 parties. Il en est de même pour les proportions de B2 et A2.

Les compositions de l'invention sont utilisées comme diélectriques pour faire des condensateurs, des transformateurs de tension ou isoler des câbles.

Les mélanges d'isomères A1 peuvent être préparés par condensation du chlorure de benzyle sur le toluène en présence de catalyseur de Friedel et Crafts. Il suffit ensuite de distiller pour les séparer.

Les mélanges d'isomères A2 peuvent être préparés par condensation du chlorure de méthylbenzyl sur le xylène en présence de catalyseurs de Friedel et Crafts. Il suffit ensuite de distiller pour les séparer.

Les oligomères de benzyltoluène A1 et B1 peuvent être fabriqués selon le procédé décrit dans le brevet européen EP 136230, les oligomères du (méthylbenzyl) xylène A2 et B2 selon le procédé décrit dans le brevet européen EP 299 867. Il suffit ensuite de les mélanger.

Pour qu'ils puissent être utilisés comme diélectriques, il est recommandé de les purifier selon une technique qui consiste à employer une terre de foulon ou d'alumine activée, soit seules, soit en mélange, selon les techniques spécifiques connues dans le secteur des liquides diélectriques.

De même, il peut être avantageux d'ajouter des stabilisants du type époxyde ou d'autre nature comme par exemple le tétraphénylétain ou des composés anthraquinoniques.

Ces adjuvants sont généralement des accepteurs d'acide chlorhydrique et sont ajoutés en quantités variables entre 0,001 et 10 %, de préférence entre 0,01 et 0,3 %.

Tous ces traitements sont connus en eux-mèmes. Selon les spécifications des diélectriques, il est parfois nécessaire d'éliminer tout le chlore organique contenu dans ces produits pour obtenir des produits à très faible teneur en chlore, c'est-à-dire moins de quelques ppm. On peut utiliser le procédé décrit dans le brevet européen EP 306 398 au nom de la demanderesse.

On ne sortirait pas du cadre de l'invention si on utilisait la composition selon l'invention en mélange avec d'autres fluides diélectriques, par exemple des tétrachlorobenzyltoluène et des chlorobenzènes ou chlorotoluènes décrits dans le brevet européen EP 8251 ou des huiles minérales habituellement utilisées dans les transformateurs.

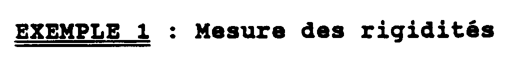

Mesures effectuées en alternatif 50 Hz, à température ambiante avec électrodes barreau ⌀ = 0,6 mm et disque ROGOWSKI ⌀ = 40 mm.

Nous avons effectué deux types d'essais :
- essais "courts" où la tension était appliquée avec une rampe de 1000 V/s ;
- essais "longs" où la tension était appliquée par paliers, avec une augmentation de 1000 V toutes les 30 secondes.

| distance inter-électrode (mm) | **essai "court"** | | | **essai "long"** | | |
|---|---|---|---|---|---|---|
| | PXE | XX01 | BT05/XX05 80/20 | PXE | XX01 | BT05/XX05 80/20 |
| 3,2 | 44,5kV | 49,9kV | | 40,2kV | 43,6kV | |
| 5 | 55,4kV | | 84,1kV | 47,8kV | 52,5kV | 57,4kV |
| 10 | 84,1kV | | >112 kV | 63,3kV | 69,5kV | 77,5kV |
| 15 | | | | 71,6kV | 82,4kV | 105,8kV |

Les chiffres correspondent à des moyennes sur 5 à 15 mesures.
---> Dans le tableau kV désigne des kilo Volts ;
---> Les valeurs peuvent varier d'environ 10 % d'une série de mesures à une autre. Mais les méthodes permettent de classer les différents diélectriques ;
---> PXE désigne un diélectrique non conforme à l'invention qui est un mélange d'isomères du phényl 1-xylyl-1-éthane de formule : 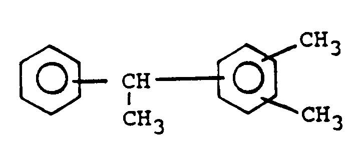
---> XX01 désigne un diélectrique de formule A2 dans laquelle q₁ et q₂ valent 0 (méthylbenzylxylène) ;
---> BT05 désigne un mélange de :
85 parties de produits de formule A1/n₁ + n₂ = 0 benzyltoluène
11 parties de produits de formule A1/n₁ + n₂ = 1 dibenzyltoluène
4 parties de produits de formule B1/S₁ₙ = 0
La quantité de B1 est donc de 4 parties pour 96 parties de A1.
---> XX05 désigne un mélange de :
85 parties de produits de formule A2/q₁ + q₂ = 0
11 parties de produits de formule A2/q₁ + q₂ = 1
4 parties de produits de formule B2/S₂q = 0
---> BT05/XX05 80/20 désigne un mélange de 80 parties de BT05 et 20 parties de XX05 ; c'est-à-dire que ce mélange, conforme à l'invention, contient :
0,8 X (85+11) parties de A1 pour
0,2 X (85+11) parties de A2
soit 76,8 parties de A1 pour 19,2 parties de A2.

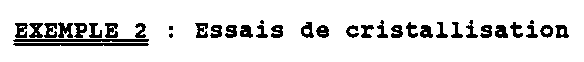

### Liquides mis en essai :

- BT06 (a) 79 parties de A1/n₁ + n₂ = 0
   16 parties de A1/n₁ + n₂ = 1
   et 5 parties de B1/Sₙ = 0
- BT01 (b) produit A1/n₁ + n₂ = 0 (benzyltoluène)
- XX01 (c) A2/q₁ + q₂ = 0
- XX05 (d) cf. Exemple 1
- mélanges :
   BT01+XX01 (e) : 25-50-75 % poids
   BT06+XX01 (f) : 25-50-75 % poids
   BT06+XX05 (g) : 25-50-75 % poids
   BT05+XX05 (h) : 5-10-15-20-25-30 % poids de XX05
- SAS-40 (i) : produit de Nippon Petrochemical à base de BT01 et Diphényléthane
- SCF 150 (j) : produit de Sybron = ditolylméthane

### Déroulement de l'essai :

**1 -** Cycles -20/-30°C sur b, c, e, f ---> rien
**2 -** Cycles -30/-40°C --->
   . après 4 jours BT01 et XX01 complètement cristallisés et servent à l'ensemencement des autres mélanges
   . après 8 jours :
      - mélanges BT06 ou BT01 + 75 % XX01 = cristallisés
      - mélanges BT06 ou BT01 + 50 % XX01 = quelques cristaux
      - mélanges BT06 ou BT01 + 25 % XX01 : rien
**3 -** Cycles -40/-45°C --->
   . après 8 jours introduction de a, g, d, i, ensemencés le lendemain
   . après 12 jours :
      - mélanges BT06 ou BT01 + 50 % XX01 : environ 1/3 cristallisés
      - BT01 + 25 % XX01 : quelques cristaux
      - BT06 : quelques cristaux dans environ 2/3 du tube
      - BT06 + XX01 (25 %) = rien
      - XX05 : rien
      - mélanges BT06 + XX05 : rien
      - SAS -40 : rien
**4 -** Cycles -45°/-50°C pendant 14 jours --->
   - BT06 : quelques cristaux sur 2/3 du tube
   - mélanges BT06 ou BT01 + 50 % XX01 : cristaux dans tout le tube
   - BT01 + 25 % XX01 : quelques cristaux
   - SAS -40 : quelques cristaux
   - BT06 + 25 % XX01 : rien
   - XX05 et mélanges BT06 + XX05 : rien
**5 -** Cycles -50°/-60°C pendant 6 jours (introduction j)
   - BT06 quelques cristaux dans tout le tube mais matrice liquide
   - mélanges avec 50 % XX01 : idem BT06
   - SCF 150 : entièrement cristallisé (après une nuit)
   - BT01 + 25 % XX01 : quelques cristaux
   - SAS -40 = quelques cristaux
   - BT06 + 25 % XX01 : rien
   - XX05 et mélanges BT06 + XX05 : rien
**6 -** Retour à cycles -40/45°C : introduction de h après 4 jours
   - SCF 150 : entièrement cristallise
   - BT01, XX01, et mélanges BT01 ou BT06 + 75 % XX01 : cristallisé
   - BT06 et mélanges BT01 ou BT06 + 50 % XX01 : fusion d'une partie des cristaux.
   - BT01 + 25 % XX01 : quelques cristaux
   - SAS -40 = quelques cristaux
   - le reste : pas de cristaux

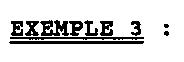

Nous avons fait une deuxième série d'essais de cristallisation, en maintenant la température pendant 43 jours à -50°C. Tous les tubes ont été "ensemencés" avec des cristaux de BT01.
- SAS -40 = cristaux dans tout le tube
- mélanges BT06/XX05 (en parties)
   - 100/0 cristaux sur 1/3 du tube
   - 95/5 cristaux sur 1/3 du tube
   - 90/10 : 2/3 du tube cristallisé
   - 85/15 : 1/3 du tube contient des cristaux
   - 80/20 : quelques rares cristaux
- mélanges BT05/XX05 (en parties)
   - 95/5 cristaux sur 1/3 du tube
   - 90/10 : quelques cristaux dans tous le tube
   - 85/15 : cristaux précipités dans le fond du tube (environ 1/4 volume)
   - 80/20 : quelques cristaux au fond
   - 75/25 : rien
70 / 30 : rien

## Revendications

1. Composition caractérisée en ce qu'elle comprend au moins un isomère ou un mélange d'isomères du benzyltoluène de formule A1 : 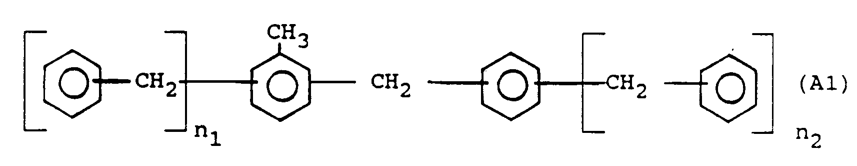 avec n₁ et n₂ = 0, 1 ou 2 sachant que n₁ + n₂ est inférieur ou égal à 3 ; et au moins un isomère ou un mélange d'isomères du (méthylbenzyl) xylène de formule A2 : 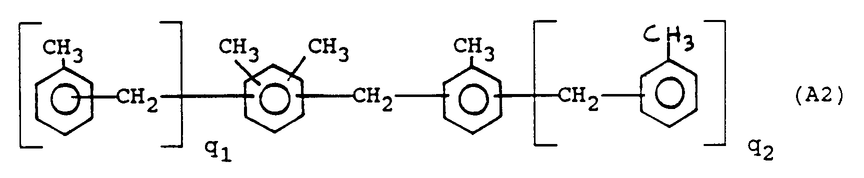 avec q₁ et q₂ = 0, 1 ou 2 sachant que q₁ + q₂ est inférieur ou égal à 3.

2. Composition selon la revendication 1, caractérisée en ce qu'en plus de l'oligomère A1 elle comprend un isomère ou un mélange d'isomères de formule B1 : 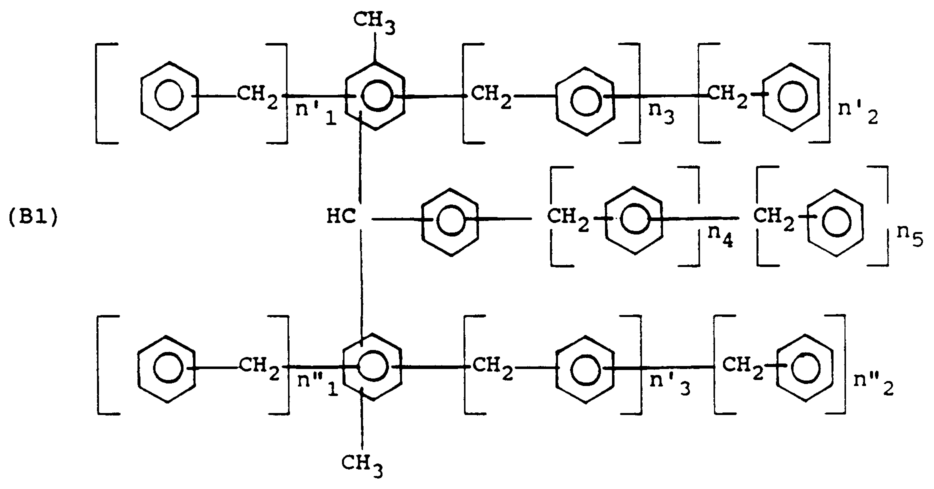 dans laquelle n'₁, n''₁ et n₄ valent 0, 1 ou 2
n'₂, n''₂, n₃, n'₃ et n₅ valent 0 et 1
sachant que n'₁ + n''₁ + n'₂ + n''₂ + n₃ + n'₃ + n₄ + n₅, qu'on désigne par S₁ₙ est inférieur ou égal à 2.

3. Composition selon la revendication 1 ou 2, caractérisée en ce qu'en plus de l'oligomère A2 elle comprend un isomère ou un mélange d'isomères de formule B2 : 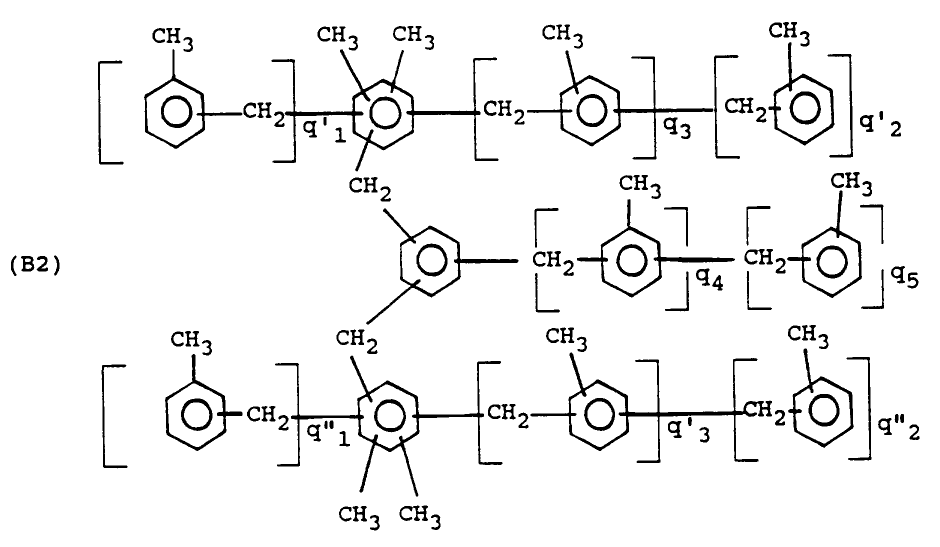 dans laquelle q'₁, q''₁ et q₄ valent 0, 1 ou 2
q'₂, q''₂, q₃, q'₃ et q₅ valent 0 et 1
sachant que q'₁ + q''₁ + q'₂ + q''₂ + q₃ + q'₃ + q₄ + q₅, qu'on désigne par S_{2q} est inférieur ou égal à 2.

4. Composition selon l'une des revendications 1 à 3, caractérisée en ce qu'elle comprend au moins 10 parties de produits A2 pour 90 parties de produit A1.

5. Composition selon la revendication 4, caractérisée en ce que la quantité de A2 varie de 15 à 90 parties pour respectivement 85 à 10 parties de A1.

6. Composition selon l'une des revendications 1 à 5, caractérisée en ce que la répartition des oligomères du benzyltoluène (produits A1) en % poids varie de :
60 à 90 % pour les isomères A1 ayant n₁ + n₂ = 0
5 à 40 % pour les isomères A1 ayant n₁ + n₂ = 1
0,5 à 8 % pour les isomères A1 ayant n₁ + n₂ = 2
Le total étant 100 %.

7. Composition selon l'une des revendications 1 à 6 caractérisée en ce que la répartition des oligomères du (méthylbenzyl) xylène (produit A2) en % poids varie de :
60 à 90 % pour les isomères A2 ayant q₁ + q₂ = 0
5 à 40 % pour les isomères A2 ayant q₁ + q₂ = 1
0,5 à 8 % pour les isomères An ayant q₁ + q₂ = 2
Le total étant 100 %

8. Composition selon l'une des revendications 1 à 7 caractérisée en ce que la quantité de produits B1 est au plus de 15 parties pour 100 parties de A1 et de préférence de 2 à 6 parties.

9. Composition selon l'une des revendications 1 à 8 caractérisée en ce que la quantité de produit B2 est au plus de 15 parties pour 100 parties de A2 et de préférence de 2 à 6 parties.

10. Application des compositions selon l'une des revendications 1 à 9 comme diélectrique notamment pour les transformateurs, les condensateurs et les câbles.

## Claims

1. Composition characterized in that it comprises at least one isomer or a mixture of isomers of benzyltoluene, of formula A1: 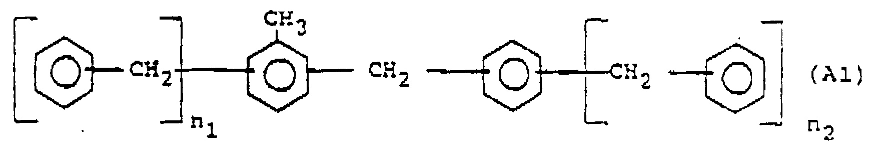 with n₁ and n₂ - 0, 1 or 2, provided that n₁ + n₂ is less than or equal to 3; and at least one isomer or a mixture of isomers of (methylbenzyl)xylene, of formula A2: 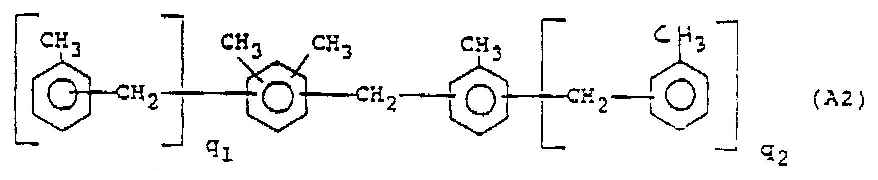 with q₁ and q₂ = 0, 1 or 2, provided that q₁ + q₂ is less than or equal to 3.

2. Composition according to Claim 1, characterized in that in addition to the oligomer A1 it comprises an isomer or a mixture of isomers of formula B1: 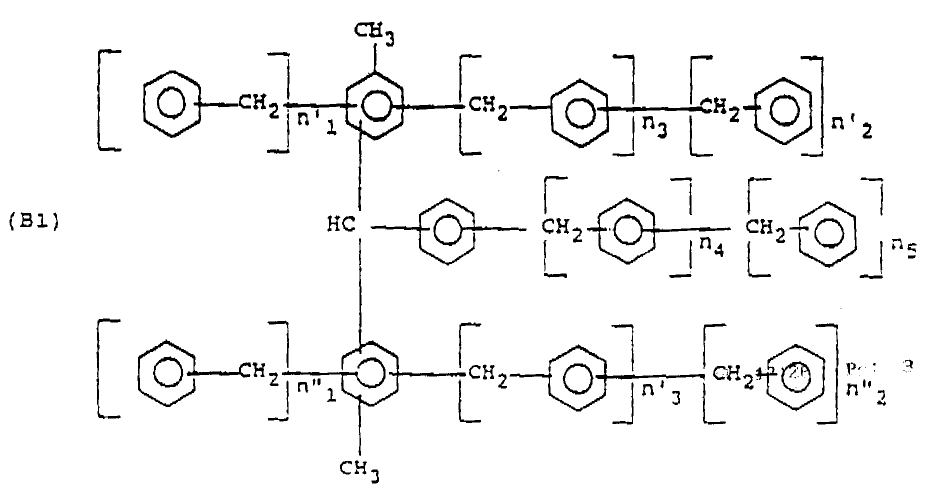 in which n'₁, n''₁ and n₄ are equal to 0, 1 or 2,
n'₂, n''₂, n₃, n'₃ and n₅ are equal to 0 or 1,
provided that n'₁ + n''₁ + n'₂ + n''₂ + n₃ + n'₃ + n₄ + n₅, which is denoted by S₁ₙ, is less than or equal to 2.

3. Composition according to Claim 1 or 2, characterized in that in addition to the oligomer A2 it comprises an isomer or a mixture of isomers of formula B2: 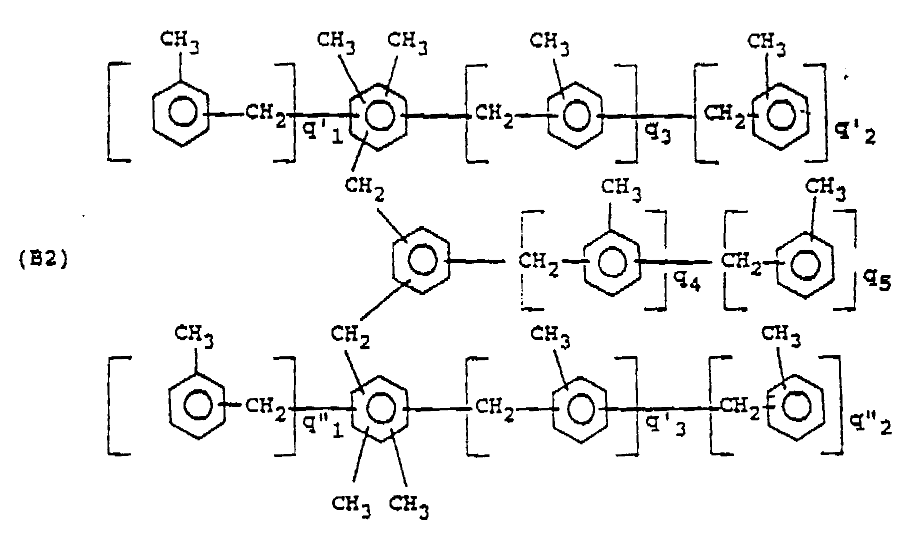 in which q'₁, q''₁ and q₄ are equal to 0, 1 or 2,
q'₂, q''₂, q₃, q'₃ and q₅ are equal to 0 or 1,
provided that q'₁ + q''₁ + q'₂ + q''₂ + q₃ + q'₃ + q₄ + q₅, which is denoted by S_{2q}, is equal to or less than 2.

4. Composition according to one of Claims 1 to 3, characterized in that it comprises at least 10 parts of products A2 per 90 parts of product A1.

5. Composition according to Claim 4, characterized in that the quantity of A2 varies from 15 to 90 parts per respectively 85 to 10 parts of A1.

6. Composition according to one of Claims 1 to 5, characterized in that the distribution of the oligomers of benzyltoluene (products A1) in weight % varies from:
60 to 90 % for the isomers A1 having n₁ + n₂ = 0
5 to 40 % far the isomers A1 having n₁ + n₂ = 1
0.5 to 8 % for the isomers A1 having n₁ + n₂ = 2,
the total being 100 %.

7. Composition according to one of Claims 1 to 6, characterized in that the distribution of the oligomers of (methylbenzyl)xylene (product A2) in weight % varies from:
60 to 90 % for the isomers A2 having q₁ + q₂ = 0
5 to 40 % for the isomers A2 having q₁ + q₂ = 1
0.5 to 8 % for the isomers A2 having q₁ + q₂ = 2,
the total being 100 %.

8. Composition according to one of Claims 1 to 7, characterized in that the quantity of products B1 is at most 15 parts per 100 parts of A1, and preferably 2 to 6 parts.

9. Composition according to one of Claims 1 to 8, characterized in that the quantity of product B2 is at most 15 parts per 100 parts of A2, and preferably 2 to 6 parts.

10. Application of the compositions according to one of Claims 1 to 9 as a dielectric, in particular for transformers, capacitors and cables.

## Patentansprüche

1. Zusammensetzung, dadurch gekennzeichnet, daß sie mindestens ein Isomer oder eine Isomerenmischung von Benzyltoluol der Formel A1 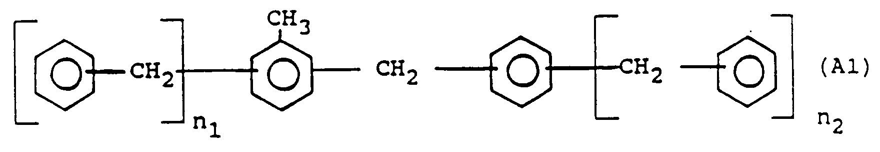 in der n₁ und n₂ = 0,1 oder 2,
vorausgesetzt, daß n₁ + n₂ kleiner oder gleich 3 ist,
und mindestens ein Isomer oder eine Isomerenmischung von (Methylbenzyl)xylol der Formel A2 enthält 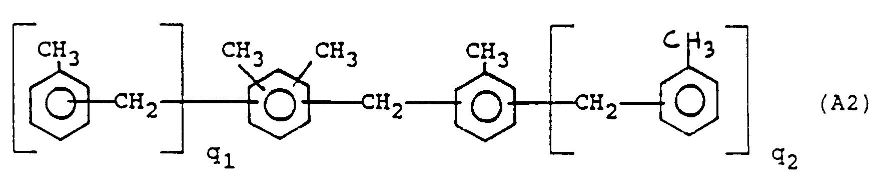 in der q₁ und q₂ = 0,1 oder 2,
vorausgesetzt, daß q₁ + q₂ kleiner oder gleich 3 ist.

2. Zusammensetzung nach Anspruch 1, dadurch gekennzeichnet, daß sie neben dem Oligomer A1 zusätzlich ein Isomer oder eine Isomerenmischung der Formel B1 enthält 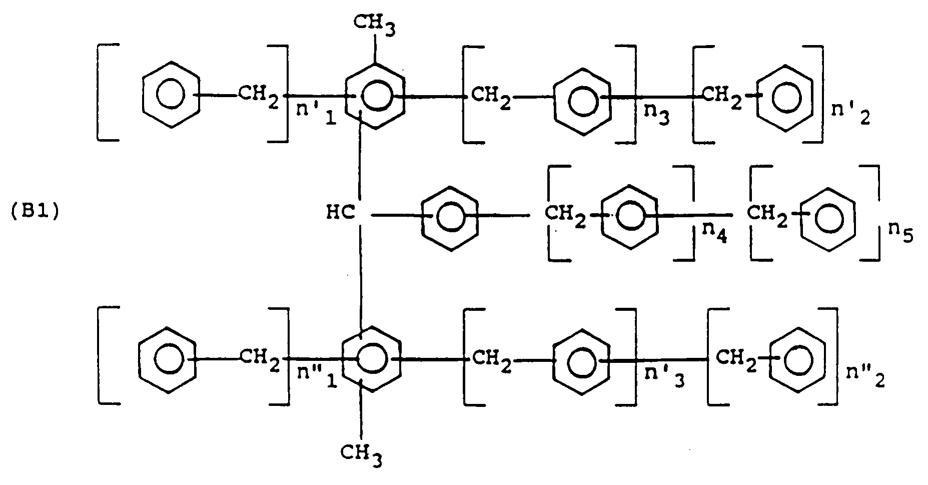 in der n'₁, n''₁ und n₄ den Wert 0,1 oder 2,
n'₂, n''₂, n₃, n'₃ und n₅ den Wert 0 und 1 haben,
vorausgesetzt daß n'₁ + n''₁ +n'2 + n''₂ + n₃ + n'₃ + n₄ + n₅,
was mit S₁ₙ bezeichnet wird, kleiner oder gleich 2 ist.

3. Zusammensetzung nach Anspruch 1 oder 2, dadurch gekennzeichnet, daß sie neben dem Oligomer A2 zusätzlich ein Isomer oder eine Isomerenmischung der Formel B2 enthält 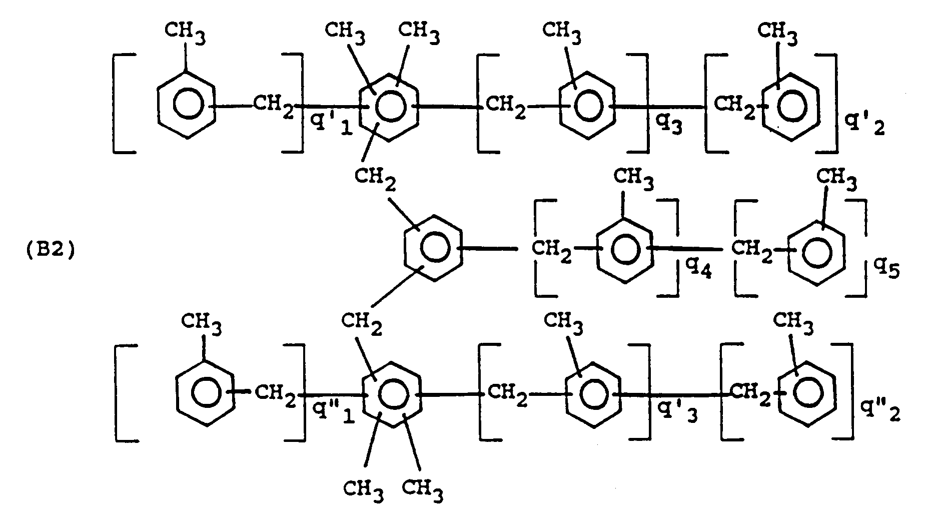 in der q'₁, q''₁ und q₄ den Wert 0,1 oder 2,
q'₂, q''₂, q₃, q'₃ und q₅ den Wert 0 und 1 haben,
vorausgesetzt, daß q'₁ + q''₁ + q'₂ + q''₂ + q₃ + q'₃ + q₄ + q₅,
was mit S_{2q} bezeichnet wird, kleiner oder gleich 2 ist.

4. Zusammensetzung nach einem der Ansprüche 1 bis 3, dadurch gekennzeichnet, daß sie mindestens 10 Teile des Produktes A2 auf 90 Teile des Produktes A1 enthält.

5. Zusammensetzung nach Anspruch 4, dadurch gekennzeichnet, daß die Menge an A2 zwischen 15 und 90 Teile auf jeweils 85 bis 10 Teile A1 beträgt.

6. Zusammensetzung nach einem der Ansprüche 1 bis 5, dadurch gekennzeichnet, daß die Verteilung der Oligomere des Benzyltoluols (Produkt A1), ausgedrückt in Gew.% zwischen
60 bis 90 %, bezogen auf die Isomeren A1 mit n₁ + n₂ = 0
5 bis 40 %, bezogen auf die Isomeren A1 mit n₁ + n₂ = 1
0,5 bis 8 %, bezogen auf die Isomeren A1 mit n₁ + n₂ = 2,
variiert. Die Summe beträgt 100 %.

7. Zusammensetzung nach einem der Ansprüche 1 bis 6, dadurch gekennzeichnet, daß die Verteilung der Oligomere des (Methylbenzyl) Xylols (Produkt A2), ausgedrückt in Gew.%, zwischen
60 bis 90 %, bezogen auf die Isomeren A2 mit q₁ +q₂ = 0
5 bis 40 %, bezogen auf die Isomeren A₂ mit q₁ +q₂ = 1
0,5 bis 8 %, bezogen auf die Isomeren A2 mit q₁ +q₂ = 2
variiert. Die Summe beträgt 100 %.

8. Zusammensetzung nach einem der Ansprüche 1 bis 7, dadurch gekennzeichnet, daß die Menge des Produktes B1 höchstens 15 Teile auf 100 Teile A1, vorzugsweise zwischen 2 bis 6 Teile beträgt.

9. Zusammensetzung nach einem der Ansprüche 1 bis 8, dadurch gekennzeichnet, daß die Menge des Produktes B2 höchstens 15 Teile auf 100 Teile A2, vorzugsweise zwischen 2 bis 6 Teile beträgt.

10. Verwendung der Mischungen nach einem der Ansprüche 1 bis 9 als Dielektrikum, insbesondere für Transformatoren, Kondensatoren und Kabel.
